# EUROPEAN PATENT APPLICATION

(11) **EP 0 911 395 A2**
(43) Date of publication of application: **28.04.1999**
(21) Application number: 98103298.0
(22) Date of filing: 25.02.1998
(51) Int. Cl.: C12N 15/06

(54) **Fused cell line and method of obtaining the same**

(30) Priority: 22.10.1997 JP 306360/97
(71) Applicant: Director-General of National Research Institute of Vegetables Ornamental Plants and Tea, Ministry of Agriculture, Forestry and, Age-gun, Mie-ken 514-23 (JP); Bio-oriented Technology Research Advancement Institution, Omiya-shi, Saitama-ken 331 (JP)
(72) Inventor: TSUji. Kenkou, Haibara-gun, Shizuoka-ken 428 (JP); Osada, Kazuhiro, Shizuoka-ken 427 (JP); Yamamoto, Mari, Shizuoka-ken 436 (JP); Kawahara, Hiroharu, Shimada-shi, Shizuoka-ken 427 (JP)
(74) Representative: VOSSIUS & PARTNER

(57) **Abstract**

Provided are a human immunocompetent fused cell line, characterized in that the mutant cell line ICLU-B derived from the human Burkitt lymphoma cell line Raji, ICLU-T derived from human T-cell lymphocytic leukemia cell line PEER, or ICLU-E derived from the human eosinophilic leukemia cell line EoL-1 is used as a parental cell line, wherein said mutant cell line is obtained by selecting an 8-azaguanine- or 6-thioguanine-resistant clone from said Burkitt lymphoma, said T-cell lymphocytic leukemia and said eosinophilic leukemia cell line, respectively, and said clone is treated in order to render it capable of growing in serum-free medium, and a method of obtaining said human immunocompetent fused cell line. The human immunocompetent fused cell line obtained by the present invention can be used to investigate food functions, novel drugs and the production thereof with the view to the elucidation of mechanisms of phenomena of the immune system such as allergies, cancers and the like, and the prevention, diagnosis and therapy of such diseases.

## Description

The present invention relates to a fused cell line and a method of obtaining the same. More specifically, the present invention relates to a human immunocompetent fused cell line which can be used to investigate food functions, novel drugs and the production thereof with a view to the elucidation of mechanisms of phenomena of the immune system such as allergies, cancers, autoimmune diseases and the like, and the prevention, diagnosis and therapy of such diseases.

The in vitro cultivation of cells separated from the human body is difficult in many cases. For example, it is difficult to infinitely grow cells, such as lymphocytes, separated from the human body in vitro. Usually, even when these cells are incubated in a culture medium, they die within from 2 weeks to several months. Some cancer cells or epithelial cells can be subcultured in vitro in a medium having some formulation. However, it is hard to obtain the same in an amount sufficient for investigation.

Further, in order to grow internal cells in vitro, it is necessary to exert some stimulus on the cells. Thus, a variety of physiologically active substances have to be added to a culture medium. However, the selection of the appropriate substances is problematic, and it takes much labor.

In recent years, in order to solve these problems, a method has been proposed in which a carcinogenic substance or the like is added to desired cells or these cells are irradiated with ultraviolet rays for mutagenization (transformation).

Such a treatment can provide cell lines (mutant strains) capable of infinite growth.

Further, a method has been also proposed in which cancer genes are injected into desired cells by a gene transfer method to cause transformation, thereby obtaining cells which can be infinitely grown.

Still further, besides the method using transformation, a method is known in which cells that produce antibodies or immunomodulating factors (lymphokines) in vivo are fused with cells to be infinitely grown to establish fused cells (hybridomas) producing antibodies or immunomodulating factors in vitro.

These conventional methods are associated, however, with the following problems.

First, with the methods using chemicals or irradiation with ultraviolet rays or the like, it takes much time, from several months to several years, to establish cell lines which are stable in the mutation obtained and the other cell functions. Further, it takes considerable time to put cell lines established to practical use. Additionally, the number of mutant strains (mutagenization efficiency) obtained relative to the number of cells treated is small. Thus, it is difficult to easily establish cell lines from objective cells.

Further, the method of obtaining cell lines through cell fusion is useful in a stable system of producing cell-derived substances such as monoclonal antibodies, lymphokines or the like. In addition, the formation of cells lines through cell fusion provides a relatively high transformation efficiency as compared to other methods. However, in the fused cell lines obtained by this method, the cell response in the various immuno-reactions which occur in vivo is said to be decreased or lost.

Accordingly, it has been quite difficult to use the in vivo cell interaction in a reproducible cell system in vitro even by these methods.

Thus, the technical problem underlying the present invention was to provide suitable cell lines to overcome the above mentioned prior art difficulties.

The solution to said technical problem is provided by the embodiments characterized in the claims.

Accordingly, the present invention relates to a human immunocompetent fused cell line, characterized in that the mutant cell line ICLU-B derived from the human Burkitt lymphoma cell line Raji, ICLU-T derived from the human T-cell lymphocytic leukemia cell line PEER or ICLU-E derived from the human eosinophilic leukemia cell line EoL-1 is used as a parental cell line, wherein said mutant cell line is obtained by selecting an 8-azaguanine- or 6-thioguanine-resistant clone from said Burkitt lymphoma, said T-cell lymphocytic leukemia and said eosinophilic leukemia cell line, respectively, and said clone is treated in order to render it capable of growing in serum-free medium.

These parental cell lines give rise to cell lines from various human immunocompetent cells at good efficiency while maintaining cell functions.

In a preferred embodiment of the present invention the treatment of said clone is carried out by incubating said clone in medium containing insulin, transferrin, ethanolamine and sodium selenite such that the fused cells which are subsequently obtained through cell fusion can be subjected to serum-free cultivation.

In a further preferred embodiment of the present invention the mutant cell line ICLU-B deposited under FERM BP-6253, ICLU-E deposited under FERM BP-6254 or ICLU-T deposited under FERM BP-6255 is used as a parental cell line.

In another embodiment the present invention relates to a method of obtaining a human immunocompetent fused cell line, which comprises forming a fused cell by using as parental cell line for human cell fusion ICLU-T derived from the human T-cell lymphocytic leukemia cell line PEER, and a human immunocompetent cell in the presence of polyethylene glycol and lecithin, preferably in a ratio of about 40:1.

In a preferred embodiment of the method of the present invention the cell line ICLU-T deposited under FERM BP-6255 is used as the parental cell line.

In a further embodiment the present invention relates to a method of obtaining a human immunocompetent cell line comprising fusing mutant cell line ICLU-B deposited under FERM BP-6253, ICLU-E deposited under FERM BP-6254 or ICLU-T deposited under FERM BP-6255 with a human immunocompetent cell.

In yet another embodiment the present invention relates to a mutant cell line selected from the group consisting of ICLU-B deposited under FERM BP-6253, ICLU-E deposited under FERM BP-6254 and ICLU-T deposited under FERM BP-6255.

Furthermore, the present invention relates to a kit comprising the cell line of the present invention.

The properties of fused cell lines greatly depend on not only the preferably human immunocompetent cells subjected to the fusion but also the preferably human parental cell lines as the other partner. Accordingly, parental cell lines have been established for preferably human cell fusion which can be used to form cell lines from a variety of preferably human immunocompetent cells at good efficiency while maintaining the cell functions. Parental cell lines have been obtained from human immunocompetent cells as follows.

A human Burkitt lymphoma cell line (Raji) and a human eosinophilic leukemia cell line (EoL-1) which are human cell lines already established were incubated in 10% FBS-ERDF medium (supplied by Kyokuto Seiyaku Kogyo K.K.) containing 6-thioguanine (final concentration 30 µg /ml) and 10% fetal bovine serum (hereinafter abbreviated as FBS). ERDF medium to which FBS is added at a concentration of 10% is described hereinafter as 10% FBS-ERDF medium. A human T-cell lymphocytic leukemia cell line (PEER) was incubated in 10% FBS-ERDF medium containing 8-azaguanine (final concentration 20 µg/ml.)

After approximately 3 weeks of incubation, grown clones were collected, and the cloning was conducted by the limiting dilution method in which a cell suspension was diluted in 10% FBS-ERDF medium such that one cell was put in one well of a 96-well culture plate. 24 to 30 hours after putting cells in a 96-well culture plate, each well of the plate was microscopically examined and every well in which two cells were observed was marked. Further 24 to 30 hours later (that is 46 to 60 hours after putting), the marked wells were microscopically examined and every well where four cells were found was again marked. 20 days after putting, the wells with two marks were microscopically examined to confirm that the number of cells has increased and to count the number of cells using a blood cell counter. The cells in the well having the largest number of cells were recloned by the same method as mentioned above.

After the parent cell line was established, the clones obtained through cloning were incubated in an ERDF medium (supplied by Kyokuto Seiyaku Kogyo K.K.) containing insulin (final concentration 10 µg/ml), transferrin (final concentration 20 µg/ml), ethanolamine (final concentration 20 µM) and sodium selenite (final concentration 25 nM) such that the fused cells obtained through the cell fusion could be subjected to serum-free culture. At this time, the incubation was conducted for approximately 2 weeks through dilution such that one cell was charged in one well of a 96-well incubation plate.

The number of cells grown in each well was counted and a part of the cells of each well was incubated in 15% FBS-ERDF medium containing aminopterin (final concentration 0.4mM), wherein the cells in the well having the largest number of cells were cultured first, the cells in the well having the second largest number of cells were cultured second, and so on. After 3 to 5 days of incubation, microscopic examination was conducted, and 3 or 4 lines of clones whose cells died were established as candidates for parent cell lines.

The cell fusion was actually conducted using said clones which became candidates for each parental cell line. As a result, the clones having the highest value (fused efficiency) calculated from the number of fused cells obtained when using 10⁵ cells of each clone as an index thereof were selected as established parental cell lines.

With respect to the thus-established three types of parental cell lines, namely, the Raji-derived parental cell line was designated a human Burkitt lymphoma derived cell line (ICLU-B), the PEER-derived parental cell line a human T-cell lymphocytic leukemia derived cell line (ICLU-T), and the EoL-1-derived parental cell line a human eosinophilic leukemia derived cell line (ICLU-E), respectively.

The cell lines ICLU-B, ICLU-E, and ICLU-T have been deposited under the provisions of the Budapest Treaty with the National Institute of Bioscience and Human Technology, Agency of Industrial Science and Technology, 1-3, Higashi 1-chome, Tsukuba-shi, Ibaraki-ken, 305-8566, Japan, on February 13, 1998 under accession number FERM BP-6253, FERM BP-6254, and FERM BP-6255, respectively.

The fused cell line of the present invention can be obtained by fusing one of these parental cell lines with a human immunocompetent cell such as a human peripheral blood lymphocyte.

The fusion of the parental cell line and the human immunocompetent cell can be conducted, e.g., in the following manner.

First, any one of the above-mentioned parental cell lines is mixed with a human immunocompetent cell. The human immunocompetent cell to be fused with the parental cell line, can be any cell having an immunological function in the human body. For example, a human lymphocyte can also be used.

The ratio of the human immunocompetent cell to the parental cell line is preferably between 1.5 and 2 times when using ICLU-B as a parental cell line, between 0.8 and 1.2 times when using ICLU-T, and between 1 and 1. 5 times when using ICLU-E.

The fusion of preferably human cells can also be conducted using, instead of preferably human immunocompetent cells, cells derived from other preferably human tissues, for example, a human cancer cell. When using a cancer cell, the tissue from which the cancer cell is derived is not limited to a particular type. For example, a gastric cancer cell or a breast cancer cell can be used. In these cases, the ratio of the cell to the parental cell line may be the same as the above-mentioned ratio.

The mixture of the parental cell line and the human immunocompetent cell is centrifuged to separate it into the supernatant and the cell pellet. Of these, the supernatant is removed. To the cell pellet is added polyethylene glycol (PEG) (fusion accelerator) which is diluted (usually to between 40 and 50%) with a basic synthetic medium.

Examples of basic synthetic media include ERDF medium, RPMI 1640 medium, and Dulbecco modified Eagle's medium (DMEM). It is also possible to use, along with this medium, fetal bovine serum (FBS) as a growth factor, or a growth factor for serum-free culture, such as insulin, transferrin, ethanolamine or sodium selenite.

Further, PEG having an average molecular weight of from 4,000 to 6,000 can be used as a fusion accelerator. In view of the fusion efficiency, PEG having an average molecular weight of 4,000 is preferred. In the case of using ICLU-T, PEG having an average molecular weight of 4,000 is especially preferred.

When ICLU-T is used as a parental cell line, a mixture of PEG and lecithin has to be used as a fusion accelerator.

When the cell fusion is conducted using ICLU-T without mixing PEG with lecithin, the disruption of the cell membrane of ICLU-T notably occurs, the number of living cells left is decreased, and the desired fused cells cannot be obtained with good efficiency.

Lecithin is a type of phospholipid which is a main component of the cell membrane. When the cell fusion using ICLU-T is conducted with a mixture of PEG and lecithin, the phospholipid which is a main component of the cell membrane is protected, making it possible to control the disruption of the cell membrane caused by PEG as much as possible.

The cell pellet containing the fusion accelerator diluted with the medium is centrifuged, and the fused cells is then selected.

The selection of the fused cells when using ICLU-B or ICLU-E as parental cell line can be conducted in a well-known selective medium (for example 15% FBS-ERDF medium) containing hypoxanthine, aminopterin (amethopterin is also possible) and thymidine.

24 to 30 hours after the fusion, a selective medium containing hypoxanthine, aminopterin and thymidine is added to the fused cells. In this case only, the concentrations of these substances in the selective medium are doubled. Thereafter, the medium having the same formulation as the initial selective medium is replaced by half every several days.

This incubation is conducted for approximately 2 weeks to be able to obtain the fused cells.

When using ICLU-T as a parental cell line, the selection of fused cells is conducted basically in the above-mentioned manner, except that the selective medium does not contain thymidine.

When thymidine is present in the selective medium when using ICLU-T as a parental cell line, growth inhibition occurs. For this reason, the addition of thymidine has to be avoided.

24 to 30 hours after the fusion, a selective medium (for example 15% FBS-ERDF medium) containing hypoxanthine and aminopterin is added to the suspension. This medium is, like the above-mentioned medium, replaced by half every several days.

The fused cells can be obtained through this incubation for approximately 2 weeks.

The fused cells obtained from the parental cells through the fusion can retain in vitro the specific cell function, preferably immuno-reaction, that the corresponding immunocompetent cells exhibit in vivo.

The human immunocompetent fused cell line of the present invention which is formed using any of ICLU-B, ICLU-T and ICLU-E as a parent cell line expresses in vitro an immunological function that the corresponding immunocompetent cells exhibit in vivo and the other properties as such.

Further, when the fused cells are formed from ICLU-T and human immunocompetent cells in the presence of polyethylene glycol and lecithin according to the method of the present invention, a cell line of fused cells which retain the immunological function in vivo as such can be established with good efficiency.

The fused cell line of the present invention can preferably be used to study in vitro the in vivo intercellular reaction.

The examples illustrate the invention:

### Example 1: Formation of a human lymphocyte cell line using ICLU-B

The parental cell line ICLU-B having a cell concentration of 1 x 10⁷ was mixed with 2 x 10⁷ human peripheral blood lymphocytes. The mixture was centrifuged, and the supernatant was separated from the cell pellet. Subsequently, this supernatant was removed.

To the remaining cell pellet was added 1 ml of 50% PEG (average molecular weight: 4,000) diluted with ERDF medium (supplied by Kyokuto Seiyaku Kogyo K.K.). Further, 9 ml of the ERDF medium were added thereto so that the total volume was adjusted to 10 ml.

The mixture was recentrifuged, and the resulting cell pellet was suspended in 50 ml of 15% FBS-ERDF medium which had been prepared such that the ERDF medium was 85% and fetal bovine serum was 15%. The suspension was added to a 96-well incubation plate such that 100 µl of the suspension were charged in each well of the plate.

On the following day of the fusion, 15% FBS-ERDF medium containing 400 µM hypoxanthine, 0.8 µM amethopterin and 32 µM thymidine was added to a 96-well incubation plate such that 100 µl of the medium were charged in each well of the plate. Then, this medium was replaced with 15% FBS-ERDF medium containing 200 µM hypoxanthine, 0.4 µM amethopterin and 16 µM thymidine by half every 2 or 3 days.

After 2 weeks of cultivation, the number of wells in which the fused cells of ICLU-B and human peripheral blood lymphocytes appeared and the fusion efficiency were measured. The results are shown in Table 1.

Further, the cell fusion was conducted in the above-mentioned manner except using ICLU-B having a cell concentration of 3 x 10⁶ and 6 x 10⁶ human peripheral blood lymphocytes. The results are also shown in Table 1.

The properties of the resulting fused cells were measured as follows.

First, an anti-B cell antibody, an anti-T cell antibody and an anti-monocyte antibody which were all fluorescent were used, and it was examined which antibody reacted with the fused cells. From the results, it could be derived whether the fused cells were B-cellular, T-cellular or monocytic.

When the fused cells were B-cellular in the examination using the antibodies, it was further investigated whether the fused cells had an antibody (Ig)-productivity.

When the fused cells were T-cellular, it was further investigated into what sub-set the fused cells could be classified. When the fused cells were monocytic, it was further investigated whether they had a phagocytic activity.

The types of the fused cells obtained by the above-mentioned two fusion procedures and the average values of the ratios and the like are shown in Table 2.

### Example 2: Formation of a human lymphocyte line using ICLU-T

ICLU-T having a cell concentration of 4 x 10⁶ was mixed with 4 x 10⁶ human peripheral blood lymphocytes. The mixture was centrifuged, and the supernatant was separated from the cell pellet. Subsequently, this supernatant was removed.

To the cell pellet was added 1 ml of 40% PEG (average molecular weight: 4,000) mixed with 1% lecithin. Further, 9 ml of ERDF medium were added thereto so that the total volume was adjusted to 10 ml.

The mixture was recentrifuged, and the resulting cell pellet was suspended in 25 ml of 15% FBS-ERDF medium which had been prepared such that the ERDF medium was 85% and fetal bovine serum was 15%. The suspension was added to a 96-well incubation plate such that 100 µl of the suspension were charged in each well of the plate.

4 days after this fusion, 15% FBS-ERDF medium containing 133 µM hypoxanthine and 0.26 µM amethopterin was added to a 96-well incubation plate such that 100 µl of the medium were charged in each well of the plate. Then, this medium was replaced with 15% FBS-ERDF medium containing 67 µM hypoxanthine and 0.13 µM amethopterin by half every 4 or 5 days.

After 2 weeks of cultivation, the number of wells in which the fused cells of ICLU-T and human peripheral blood lymphocytes appeared and the fusion efficiency were measured. The results are shown in Table 1.

Further, the cell fusion was conducted in the above-mentioned manner except using ICLU-T having a cell concentration of 5 x 10⁶ and 5 x 10⁶ human peripheral blood lymphocytes. The results are also shown in Table 1.

The properties of the resulting fused cells were measured in the same manner as in Example 1. The results are shown in Table 2.

### Example 3: Formation of a human lymphocyte cell line using ICLU-E

ICLU-E having a cell concentration of 1 x 10⁷ was mixed with 1 x 10⁷ human peripheral blood lymphocytes. The mixture was centrifuged, and the supernatant was separated from the cell pellet. Subsequently, this supernatant was removed.

To the remaining cell pellet was added 1 ml of 40% PEG (average molecular weight: 4,000) diluted with ERDF medium. Further, 9 ml of ERDF medium (supplied by Kyokuto Seiyaku Kogyo K.K.) were added thereto so that the total volume was adjusted to 10 ml.

The mixture was recentrifuged, and the resulting cell pellet was suspended in 50 ml of 15% FBS-ERDF medium which had been prepared such that the ERDF medium was 85% and fetal bovine serum was 15%. The suspension was added to a 96-well incubation plate such that 100 µl of the suspension were charged in each well of the plate.

On the following day of this fusion, 15% FBS-ERDF medium containing 400µM hypoxanthine, 0.8 µM amethopterin and 32 µM thymidine was added to a 96-well incubation plate such that 100 µl of the medium were charged in each well of the plate. Then, this medium was replaced with 15% FBS-ERDF medium containing 200 µM hypoxanthine, 0.4 µM amethopterin and 16 µM thymidine by half every 2 or 3 days.

After 2 weeks of the cultivation, the number of wells in which the fused cells of ICLU-E and human peripheral blood lymphocytes appeared and the fusion efficiency were measured. The results are shown in Table 1.

Further, the cell fusion was conducted in the above-mentioned manner except using ICLU-E having a cell concentration of 7 x 10⁶ and 7 x 10⁶ human peripheral blood lymphocytes. The results are shown in Table 1.

The properties of the resulting fused cells were measured in the same manner as in Example 1. The results are shown in Table 2.

**Table 1**

| | Type of parental cell line | Number of cells of parental cell | Number of wells in which fused cells appeared | Fusion efficiency (per 10⁵ cells of the parental cell line) |
|---|---|---|---|---|
| Example 1 | ICLU-B | 1 X 10⁷ | 130 | 1.3 |
| | | 3 X 10⁶ | 30 | 1.0 |
| Example 2 | ICLU-T | 4 X 10⁶ | 8 | 0.2 |
| | | 5 X 10⁶ | 9 | 0.18 |
| Example 3 | ICLU-E | 1 X 10⁷ | 48 | 0.48 |
| | | 7 X 10⁶ | 15 | 0.21 |

**Table 2**

| | Parental cell line | Type of cells of the fused cell line obtained and the ration thereof | Remarks |
|---|---|---|---|
| Example 1 | ICLU-B | B cells: 72.3% | IgG, IgM and IgE are produced. |
| | | T cells: 18.2% | |
| | | Monocytes: 9.5% | A phagocytic activity is exhibited. |
| Example 2 | ICLU-T | B cells: 11.1% | Helper-type |
| | | T cells: 77.8% | |
| | | Monocytes: 11.1% | |
| Example 3 | ICLU-E | B cells: 20% | Ig is not produced. |
| | | T cells: 57.1% | A phagocytic activity is exhibited |
| | | Monocytes: 22.9% | |

Table 1 shows that any of the parental cell lines could provide the fused cells with the human peripheral blood lymphocytes with high efficiency through the incubation for 2 weeks.

Further, Table 2 reveals the following.

First, it is evident that any of the three parental cell lines can form cell lines of various immunocompetent cells in vivo.

When ICLU-B is used as the parental cell line for the cell fusion, any of the three immunocompetent cells can be obtained. Mainly, the B cells are provided. The fused cell line of the B cells obtained from ICLU-B has an Ig-productivity. Thus, it is clear that this cell line retains the properties of B lymphocytes which are antibody-productive cells among peripheral blood lymphocytes subjected to the fusion.

Further, when ICLU-T is used as the parental cell line for the cell fusion, the fused cells of the T cell type are mainly obtained. These fused cells of the T cell type are of the helper type. Accordingly, it could be shown that these fused cells retain the properties of the T lymphocytes among the peripheral blood lymphocytes subjected to the cell fusion.

However, when ICLU-E is used as the parental cell line for the cell fusion, the resulting fused cells chiefly exhibit the properties of the T cell type. However, as compared to the case of using ICLU-B or ICLU-T as the parental cell line, a large number of monocytic fused cells are obtained.

Since this fused monocytic line has a phagocytic activity, it is clear that this cell line retains the properties of the monocytes.

### Example 4: Examination of a fusion accelerator when cell fusion is conducted using the parental cell line ICLU-T

ICLU-T cells and human peripheral blood lymphocytes were mixed such that the ratio of these cells was 1:1 (specific value is shown in Table 3). This mixture was centrifuged, and the supernatant was separated from the cell pellet. This supernatant was then removed.

To the cell pellet was then added 1 ml of a mixture of 1% lecithin and 40% PEG (average molecular weight: 4,000) prepared using ERDF medium (supplied by Kyokuto Seiyaku Kogyo K.K.) as a basic medium and 1% lecithin. Further, 9 ml of ERDF medium were added thereto so that the total volume was adjusted to 10 ml.

The mixture was recentrifuged, and the resulting cell pellet was suspended in 50 ml of 15% FBS-ERDF medium which had been prepared such that the ERDF medium was 85% and FBS was 15%. The suspension was added to a 96-well incubation plate such that 100 µl of the suspension were charged in each well of the plate.

4 days after the fusion, 15% FBS-ERDF medium containing 133 µM hypoxanthine and 0.26 µM amethopterin was added to a 96-well incubation plate such that 100 µl of the medium were charged in each well of the plate. Then, this medium was replaced with 15% FBS-ERDF medium containing 67 µM hypoxanthine and 0.13 µM amethopterin by half every 4 or 5 days.

After 2 weeks of cultivation, the number of wells in which the fused cells of ICLU-T and human peripheral blood lymphocytes appeared and the fusion efficiency were measured. The results are shown in Table 3.

On the other hand, as a control, the cell fusion was conducted in the above-mentioned manner except using 40% PEG (average molecular weigh t: 4,000) diluted with lecithin-free ERDF medium. The results are also shown in Table 3.

**Table 3**

| Formulation of fused accelerator | Number of cells subjected to fusion | Number of wells in which fused cells appeared | Fusion efficiency* |
|---|---|---|---|
| PEG + lecithin | 4 x 10⁶ | 8 | 0.20 |
| | 5 x 10⁶ | 9 | 0.18 |
| PEG alone | 1 x 10⁷ | 2 | 0.02 |
| | 5 x 10⁶ | 0 | 0 |
| | 7 x 10⁶ | 1 | 0.01 |

| | | | |
|---|---|---|---|
| * per 10⁵ parent cells | | | |

Table 3 reveals that when PEG alone is used as a fusion accelerator, no fused cell is obtained or the fusion efficiency is quite low even when fusion cells are provided. It is clear that when both PEG and lecithin are used, the fused cells can be obtained with good efficiency.

The above-mentioned results show that when the cell fusion is conducted using ICLU-T as the parental cell line, both PEG and lecithin have to be added as fusion accelerator.

## Claims

1. A human immunocompetent fused cell line, characterized in that the mutant cell line ICLU-B derived from the human Burkitt lymphoma cell line Raji, ICLU-T derived from the human T-cell lymphocytic leukemia cell line PEER, or ICLU-E derived from the human eosinophilic leukemia cell line EoL-1 is used as a parental cell line, wherein said mutant cell line is obtained by selecting an 8-azaguanine- or 6-thioguanine-resistant clone from said Burkitt lymphoma, said T-cell lymphocytic leukemia and said eosinophilic leukemia cell line, respectively, and said clone is treated in order to render it capable of growing in serum-free medium.

2. The cell line of claim 1, wherein the treatment of said clone is carried out by incubating said clone in medium containing insulin, transferrin, ethanolamine and sodium selenite such that the fused cells which are subsequently obtained through cell fusion can be subjected to serum-free cultivation.

3. The cell line of claim 1 or 2, wherein the mutant cell line ICLU-B deposited under FERM BP-6253, ICLU-E deposited under FERM BP-6254 or ICLU-T deposited under FERM BP-6255 is used as a parental cell line.

4. A method of obtaining a human immunocompetent fused cell line, which comprises forming a fused cell by using as parental cell line for human cell fusion ICLU-T derived from the human T-cell lymphocytic leukemia cell line PEER and a human immunocompetent cell in the presence of polyethylene glycol and lecithin.

5. The method of claim 4, wherein polyethylene glycol and lecithin are used in a ratio of about 40:1.

6. The method of claim 4 or 5, wherein the cell line ICLU-T deposited under FERM BP-6255 is used as the parental cell line.

7. A method of obtaining a human immunocompetent cell line comprising fusing mutant cell line ICLU-B deposited under FERM BP-6253, ICLU-E deposited under FERM BP-6254 or ICLU-T deposited under FERM BP-6255 with a human immunocompetent cell.

8. A mutant cell line selected from the group consisting of ICLU-B deposited under FERM BP-6253, ICLU-E deposited under FERM BP-6254 and ICLU-T deposited under FERM BP-6255.

9. A kit comprising the cell line of any one of claims 1 to 3 or 8.
